# FASCICULE DE BREVET EUROPEEN

(11) **EP 1 465 893 B1**
(45) Date de publication et mention de la délivrance du brevet: **28.02.2007**
(21) Numéro de dépôt: 03712202.5
(22) Date de dépôt: 03.01.2003
(51) Int. Cl.: C07D 471/08, A61K 31/439, A61P 25/00

(54) **DERIVES DE 5-(PYRIDIN-3-YL)-1-AZABICYCLO (3.2.1) OCTANE, LEUR PREPARATION ET LEUR APPLICATION EN THERAPEUTIQUE**
(5-(PYRIDIN-3-YL)-1-AZABICYCLO(3.2.1) OCTAN DERIVATE, DEREN HERSTELLUNG UND DEREN VERWENDUNG IN THERAPEUTIKA"
DERIVATIVES OF 5-(PYRIDIN-3-YL)-1-AZABICYCLO (3.2.1) OCTANE, THE PREPARATION THEREOF AND THE APPLICATION OF SAME IN THERAPEUTICS

(30) Priorité: 07.01.2002 FR 0200109
(43) Date de publication de la demande: 13.10.2004
(73) Titulaire: Sanofi-Aventis, 75013 Paris (FR)
(72) Inventeur: GALLI, Frédéric, F-92420 Vaucresson (FR); LECLERC, Odile, F-91300 Massy (FR); LOCHEAD, Alistair, F-94220 Charenton le Pont (FR); NEDELEC, Alain, 75015 Paris (FR)
(74) Mandataire: Ludwig, Jacques
(86) Numéro de dépôt international: PCT/FR2003/000004
(87) Numéro de publication internationale: WO 2003/057697

(56) Documents cités:
- WO-A-00/34279
- WO-A-00/34284
- US-A- 5 817 679

## Description

La présente invention a pour objet des composés qui sont des ligands des récepteurs nicotiniques, et qui sont utiles dans le traitement ou la prévention des désordres liés à un dysfonctionnement des récepteurs nicotiniques, notamment au niveau du système nerveux central.

Les composés de la présente invention répondent à la formule générale (I) 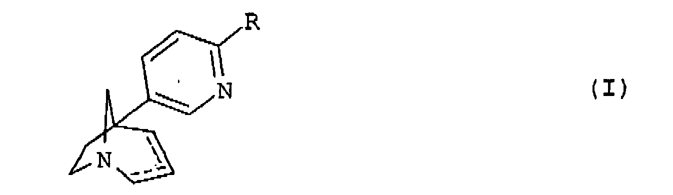 dans laquelle R représente un atome d'halogène ou un groupe (C₃-C₆)cycloalkyle ou un groupe phényle substitué par un ou plusieurs groupes choisis parmi un atome d'halogène, ou un groupe (C₁-C₆) alkyle, (C₁-C₆) alcoxy, nitro, amino, (C₁-C₃)dialkylamino, trifluorométhyle, trifluorométhoxy, cyano, hydroxy, acétyle ou méthylènedioxy, ou un groupe pipéridinyle, ou morpholin-4-yle, ou pyrrolidin-1-yle, ou azétidin-1-yle, ou azépin-1-yle, ou pyridinyle, ou quinoléinyle, ou thiényle, ou pyrazinyle, ou furyle, ou benzofuryle, ou benzothiényle, ou indolyle, ou pyrimidinyle, ou isoxazolyle, ou phénoxazinyle, ou phénoxathiinyle, ou dibenzothiényle, ou dibenzofuryle, ou pyrrolyle ou naphtyle, chacun de ces groupes pouvant éventuellement être substitué par un ou plusieurs groupes choisis parmi les atomes d'halogènes, les groupes (C₁-C₆) alkyle, (C₁-C₆) alcoxy, trifluorométhoxy, trifluorométhyle, nitro, cyano, hydroxy, amino, (C₁-C₃)dialkylamino ou (C₃-C₈)cycloalkylamino; les composés suivants étant exclus:
- 5-(2-bromopyridin-5-yl)-1-azabicyclo[3.2.1]oct-3-ane
- 5-(2-chloropyridin-5-yl)-1-azabicyclo[3.2.1]oct-3-ane
- 5-(2--fluoropyridin-5-yl)-1-azabicyclo[3.2.1]oct-3-ane.

Les composés exclus ci-dessus sont décrits dans le document WO95/03306 comme composés arthropodicides.

Parmi les deux liaisons carbone-carbone représentées par 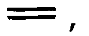 l'une est simple et l'autre peut être simple ou double. Par ailleurs, l'atome de carbone en position 5 est asymétrique, des sorte que les composés peuvent exister sous forme de deux énantiomères ou de mélanges de ces derniers.

Les composés de l'invention peuvent exister à l'état de bases ou de sels d'addition à des acides.

Un sous ensemble de composés préférés est celui des composés de formule générale (I) dans laquelle R représente soit un atome d'halogène ou un groupe phényle substitué par un ou plusieurs groupes choisis parmi les atomes d'halogènes et les groupes (C₁-C₆) alkyle, (C₁-C₆) alcoxy, nitro, amino, trifluorométhyle, cyano, hydroxy, acétyle ou méthylènedioxy, soit un groupe pyridinyle, soit un groupe thiényle, soit un groupe indolyle, soit un groupe pyrimidinyle éventuellement substitué par par un ou plusieurs groupes (C₁-C₆) alcoxy.

Les composés de formule générale (I) peuvent être préparés par un procédé illustré par le schéma qui suit.
On fait réagir le 3-oxo-1,4-azabicyclo [2.2.2] octane, de formule (II), avec un dérivé de pyridine de formule générale (III), dans laquelle R est tel que défini ci-dessus et W représente un atome d'halogène.
On peut ainsi effectuer une réaction de condensation entre le 3-oxo-1-azabicyclo[2.2.2]octane et le dérivé lithié des composés de formule générale (III) obtenus par échange halogène - métal avec un dérivé alkyllithium.
On obtient des composés de formule générale (IV) qui, lorsqu'ils sont traités en milieu acide à chaud conduisent aux composés de formule générale (I) dans laquelle l'une des deux liaisons carbone-carbone représentées par 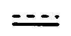 est double. L'hydrogénation catalytique de la double liaison conduit aux composés de formule générale (I) dont toutes les liaisons du cycle d'azabicyclooctane sont saturées.

Le 3-oxo-1-azabicyclo[2.2.2]octane est disponible dans le commerce.
Les composés de formule générale (III) sont disponibles dans le commerce ou sont accessibles par des méthodes décrites dans la littérature.

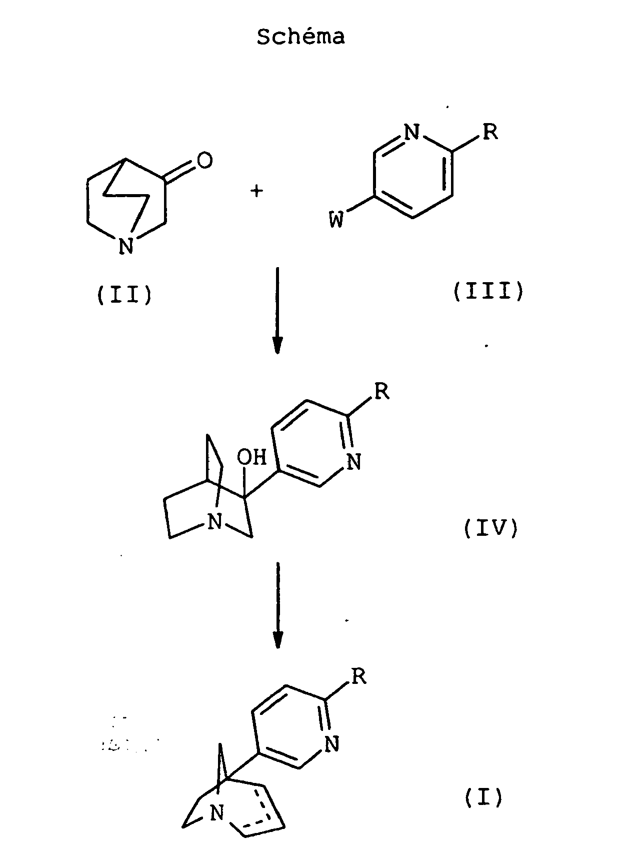

Pour certains composés, les substituants R ne sont pas présents dans le composé de départ de formule générale (III) ; selon leur nature, ces substituents peuvent être introduits sur le composé final de formule générale (I). Ainsi, par exemple des composés de formule générale (I) dans laquelle R représente un groupement aryle peuvent être préparés à partir des composés correspondants, dans la formule desquels R représente un atome d'halogène, selon toutes méthodes connues, telles qu'un couplage de type Suzuki en présence d'un acide boronique et d'un catalyseur au palladium, par exemple le tétrakistriphénylphosphine palladium, ou un couplage de Stille avec les réactifs appropriés.

Les exemples qui vont suivre illustrent la préparation de quelques composés de l'invention. Les microanalyses élémentaires, et les spectres I.R. et R.M.N. confirment les structures des composés obtenus.
Les numéros indiqués entre parenthèses dans les titres des exemples correspondent à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1).

### Bromhydrate de 5-(2-phénylpyridin-5-yl)-1-azabicyclo[3.2.1]oct-3-ène (2:1).

### 1.1. 5-Bromo-2-phényl-pyridine

Dans un ballon tricol de 500 ml on introduit successivement 30 g (0,127 mol) de 2,5-dibromopyridine en suspension dans 100 ml de toluène, 15,4 g (0,127 mol) d'acide phénylboronique, 4,4 g (0,0038 mol) de tétrakis-(triphénylphosphine)-palladium, 90 ml d'une solution aqueuse 2M de carbonate de sodium et 4 ml d'éthanol, et on chauffe le mélange à 90°C pendant 22 h.
On le décante, on lave la phase organique avec 100 ml d'eau, on la sèche, on la concentre sous pression réduite et on purifie le résidu par chromatographie sur colonne de gel de silice en éluant avec un mélange 30/70 de cyclohexane et dichlorométhane.
On obtient 22,4 g de cristaux.
Point de fusion : 69-72°C.

### 1.2. 3-hydroxy-3-(2-phénylpyridin-S-yl)-1-azabicyclo-[2.2.2]octane.

Dans un ballon tricol de 100 ml on introduit 2,5 g (0,0107 mole) de 5-bromo-2-phényl-pyridine en solution dans 40 ml d'éther éthylique et on refroidit le milieu réactionnel à -60°C pour ajouter, goutte à goutte, en 10 min, 5,6 ml (0,0139 mole) d'une solution de n-butyllithium à 2,5 M dans l'hexane, et on maintient a température à -70°c pendant 1 h.
On ajoute 1,34 g(0.0107 mole) de 1-azabicyclo [2.2.2] octane-3-one en solution dans 20 ml de tétrahydrofurane en 10 min, on agite le mélange pendant 30 min à -70°C puis pendant 4 h à température ambiante.
On hydrolyse le milieu réactionnel par addition de 100 ml de méthanol et on le concentre sous pression réduite. On reprend le résidu par 100 ml d'une solution aqueuse saturée de chlorure d'ammonium et on extrait la phase aqueuse par du chloroforme. On sèche les phases organiques, on les concentre sous pression réduite et on purifie le résidu par chromatographie sur colonne de gel de silice en éluant avec un mélange 90/10/1 de chloroforme, méthanol et ammoniaque. On obtient 0,8 g de cristaux.
Point de fusion : 214°C.

### 1.3. Bromhydrate de 5-(2-phénylpyridin-5-yl)-1-azabicyclo [3.2.1] oct-3-ène (2:1).

Dans un ballon tricol de 25 ml on introduit 0,8 g (2,85 mmoles) de 3-hydroxy-3-(2-phénylpyridin-5-yl)-1-azabicyclo[2.2.2]octane puis 10 ml d'acide méthanesulfonique et on chauffe le mélange à 180°C pendant 24 h.
On le verse sur de la glace, on alcalinise par addition d'une solution aqueuse concentrée d'hydroxyde de sodium, on extrait la phase aqueuse par du chloroforme, on sèche la phase organique et on la concentre sous pression réduite. On purifie le résidu par chromatographie sur colonne de gel de silice en éluant avec un mélange 98/2/0,2 de chloroforme, méthanol et ammoniaque.
On obtient 0,25 g de produit dont on fait le dibromhydrate par addition d'une solution d'acide bromhydrique 5,7 M dans l'acide acétique.
On obtient 0,22 g de dibromhydrate.
Point de fusion : 273-274°C.

### Exemple 2 (Composé N° 2).

### Bromhydrate de 5-(2-phénylpyridin-5-yl)-1-azabicyclo [3.2.1] octane (2:1).

Dans un flacon de Parr de 250 ml on introduit 0,14 g (0,33 mmole) de dibromhydrate de 5- (2-phénylpyridin-5-yl) -1-azabicyclo[3.2.1]oct-3-ène en solution dans 20 ml de méthanol et on ajoute 0,14 g de palladium à 10% adsorbé sur charbon. On soumet ensuite le milieu réactionnel à une pression de 0,35 Mpa d'hydrogène sous agitation pendant 5 h.
On recueille le catalyseur par filtration sur terre d'infusoires et on concentre le solvant sous pression réduite.
On obtient 0,058 g de produit.
Point de fusion : 272-277°C.

### Exemple 3 (Composé N°8).

### Ethanedioate de 5-[2-(3-méthylphényl)pyridin-5-yl)-1-azabicyclo[3.2.1]oct-3-ène (1:1).

### 3.1. 3-Hydroxy-3-(2-bromopyridin-5-yl)-1-azabicyclo [2.2.2] octane.

Dans un ballon tricol de 2000 ml on introduit 27,6 g (0,116 mole) de 2,5-dibromopyridine dans 1000 ml d'éther éthylique, on refroidit le milieu réactionnel à -67°C et on ajoute 56 ml (0,140 mole) d'une solution 2,5 M de butyllithium dans l'hexane, goutte à goutte, en 10 min. On laisse agiter le milieu à -67°C pendant 45 min avant d'ajouter 14,5 g (0,116 mole) de 1-azabicyclo[2.2.2]octane-3-one en solution dans 150 ml d'éther éthylique en 45 min et on laisse agiter à -67°C pendant 3 h.
On ajoute 300 ml d'une solution aqueuse saturée de chlorure d'ammonium, puis 200 ml d'une solution aqueuse concentrée d'hydroxyde de sodium, on extrait la phase aqueuse par du chloroforme, on sèche les phases organiques et on les concentre sous pression réduite. On purifie le résidu par chromatographie sur colonne de gel de silice en éluant avec un mélange 95/5/0,5 puis 80/15/1,5 de chloroforme, méthanol et ammoniaque .
On obtient 19,7 g de produit sous forme de solide amorphe.

3.2. 5-(2-bromopyridin-5-yl)-1-azabicyclo[3.2.1]oct-3-ène. Dans un ballon tricol de 100 ml on introduit 9,4 g (0,033 mole) de 3-hydroxy-3-(2-bromopyridin-5-yl)-1-azabicyclo_ [2.2.2]octane et 35 ml d'acide sulfurique concentré et on chauffe le mélange à 190°C pendant 1h45.
On le refroidit, on le verse sur 400 ml d'une solution aqueuse glacée d'hydroxyde de sodium, on extrait la phase aqueuse par du chloroforme, on sèche les phases organiques et on les évapore sous pression réduite. On purifie le résidu par chromatographie sur colonne de gel de silice en éluant avec un mélange 90/10/1 de chloroforme, méthanol et ammoniaque .
On obtient 3,9 g de produit sous forme de solide jaune pâle.
Point de fusion : 73-75°C.

### 3.3. Ethanedioate de 5-[2-(3-méthylphényl)pyridin-5-yl)-1-azabicyclo[3.2.1]oct-3-ène (1:1).

Dans un tube de 10 ml on introduit successivement 0,2 g (0,75 mmole) de 5-(2-bromopyridin-5-yl)-1-azabicyclo-[3.2.1]oct-3-ène, 3 ml de toluène, 0,7 ml d'une solution aqueuse 2M de carbonate de sodium, 0,147g (1,05 mmole)d'acide 3-méthylbenzèneboronique, 0,042 g (0,04 mmole) de tétrakis(triphénylphosphino)palladium et 0,7 ml d'éthanol, et on chauffe le mélange à 100°C pendant 15 h. eures.
On élimine la phase aqueuse par décantation et on extrait le brut sur colonne de résine Dowex® par lavages successifs par du méthanol puis du chloroforme avant d'éluer par une solution d'ammoniaque. On purifie le résidu par chromatographie sur colonne de gel de silice en éluant avec un mélange 90/10/1 de chloroforme, méthanol et ammoniaque. On obtient ainsi 0,167 g de produit sous forme d'huile, que l'on met en solution dans 2 ml d'alcool isopropylique pour faire un éthanedioate par addition de 0,051 g (0,057 mmole) d'acide éthanedioïque en solution dans l'alcool isopropylique. On obtient 0,188 g de produit cristallisé.
Pf: 173-174°C.

### Exemple 4 (Composé N°26).

### Bromhydrate de 5-[2-(3-fluorophényl)pyridin-5-yl)-1-azabicyclo[3.2.1]octane 2:1.

Dans un flacon de Parr de 250 ml on introduit 0,18 g (0,51 mmole) d'éthanedioate de 5-[2-(3-fluorophényl)pyridin-5-yl)-1-azabicyclo[3.2.1]oct-3-ène en solution dans 20 ml de méthanol, on ajoute 0,36 g de palladium à 10% adsorbé sur charbon et on soumet le milieu réactionnel à une pression de 0,42 Mpa d'hydrogène sous agitation à 45°C pendant 6 h. On recueille le catalyseur par filtration sur terre d'infusoires®, on concentre le filtrat sous pression réduite, on reprend le résidu dans 10 ml d'une solution aqueuse d'hydroxyde de sodium N et on extrait la phase aqueuse par du chloroforme et on purifie le brut par chromatographie sur colonne de gel de silice en éluant avec un mélange 80/20/2 de chloroforme, méthanol et ammoniaque. On obtient 0,085 g de produit dont on fait le dibromhydrate par addition de 0,107 ml d'une solution d'acide bromhydrique à 33% dans l'acide acétique. On obtient 0,097 g de cristaux.
Point de fusion : 98-100°C.

Le tableau qui suit illustre les structures chimiques et les propriétés physiques de quelques composés de l'invention. Dans la colonne "R", "(+)" désigne l'énantiomère dextrogyre et "(-)" l'énantiomère lévogyre ; les composés sans mention dans cette colonne sont des racémates. Dans la colonne " ‗ ", le nombre indiqué correspond à la position de la double liaison dans le cas d'un 1-azabiclo-octène, et "-" désigne un hétérocycle saturé. Dans la colonne "Sel", "-" désigne un composé à l'état de base, "Hbr" désigne un bromhydrate et "ox." désigne un oxalate. Les rapports molaires acide:base sont indiqués en regard. Dans la colonne "F(°C)", "(d)" désigne un point de fusion avec décomposition.

**Tableau**

| | | | | |
|---|---|---|---|---|
| 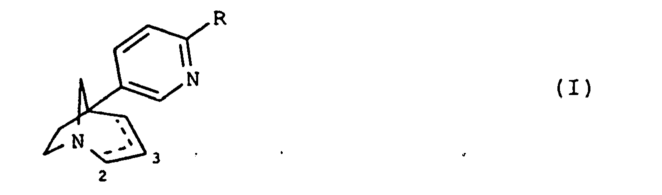 | | | | |

| N° | R | 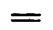 | Sel | F(°C) |
|---|---|---|---|---|
| 1 | C₆H₅ | 3 | HBr 2:1 | 273-274 |
| 2 | C₆H₅ | - | HBr 2:1 | 272-277 |
| 3 | C₆H₅ | 2 | HBr 2:1 | 297-305 |
| 4 | 2,4-(OCH₃)₂-5-pyrimidinyl | 2 | HBr 2:1 | 340 (d) |
| 5 | 3,4-(OCH₃)₂-C₆H₃ | 3 | HBr 2:1 | 261-262 |
| 6 | 3,4-(OCH₃)₂-C₆H₃ | - | HBr 2:1 | 234-236 |
| 7 | 2-F-C₆H₄ | 3 | ox. 1:1 | 157-158 |
| 8 | 3-CH₃-C₆H₄ | 3 | ox. 1:1 | 173-174 |
| 9 | 3-F-C₆H₄ | 3 | ox. 1:1 | 163-164 |
| 10 | 3-NO₂-C₆H₄ | 3 | ox. 1:1 | 183-184 |
| 11 | 3-CF₃-C₆H₄ | 3 | ox. 1:1 | 156-157 |
| 12 | 4-CH₃-C₆H₉ | 3 | ox. 1:1 | 213-215 |
| 13 | 3-Thienyl | 3 | ox. 1:1 | 189-190 |
| 14 | 3,4-OCH₂O-C₆H₃ | 3 | ox. 1: 1 | 201-202 |
| 15 | 4-Cl-C₆H₄ | 3 | ox. 1:1 | 201-203 |
| 16 | 3-CH₃CO-C₆H₄ | 3 | ox. 1:1 | 155-156 |
| 17 | 3-Pyridinyl | 3 | ox. 1:1 | 183-184 |
| 18 | 5-Indolyl | 3 | ox. 1:1 | 253-254 |
| 19 | 4-CH₃O-C₆H₄ | 3 | ox. 1:1 | 205-207 |
| 20 | 3, 5-(CH₃)₂-C₆H₃ | 3 | ox. 1: 1 | 192-193 |
| 21 | 4-Pyridinyl | 3 | ox. 1:1 | 172-174 |
| 22 | 4-CH₃O-C₆H₄ | - | HBr 2:1 | 246-247 |
| 23 | 4-CH₃-C₆H₄ | - | HBr 2:1 | 295-297 |
| 24 | 3-CH₃-C₆H₄ | - | HBr 2:1 | 284-287 |
| 25 | 3,5-(CH₃)₂-C₆H₃ | - | HBr 2:1 | 250-254 |
| 26 | 3-F-C₆H₄ | - | HBr 2:1 | 98-100 |
| 27 | 3-Thienyl | - | HBr 2:1 | 193-196 |
| 28 | 3,4-OCH₂O-C₆H₃ | - | HBr 2:1 | 260-263 |
| 29 | 2-F-C₆H₄ | - | HBr 2:1 | 266-269 |
| 30 | 3-Pyridinyl | - | HBr 3:1 | 256-260 |
| 31 | 4-Pyridinyl | - | HBr 2:1 | 249-253 |
| 32 | 3-NO₂-C₆H₄ | - | HBr 3:1 | 264-267 |
| 33 | 3-CF₃-C₆H₄ | - | HBr 2:1 | 218-221 |
| 34 | Br | 3 | HBr 2:1 | 234-236 |
| 35 | Br | 2 | HBr 2:1 | >350 |
| 36 | 4-Piperidinyl | - | HBr 3:1 | 289-292 |
| 37 | 3-Piperidinyl | - | HBr 3:1 | 261-265 |
| 38 | 4-CH₃O-C₆H₄ (+) | - | - | 125-129 |
| 39 | 4-CH₃O-C₆H₄ (-) | - | - | 125-129 |
| 40 | 3-F-C₆H₄ (+) | - | - | 68-70 |
| 41 | 3-F-C₆H₄ (-) | - | - | 68-70 |
| 42 | 2-Thienyl | - | HBr 2:1 | 251 (d) |
| 43 | 2-Thienyl | 3 | HBr 2:1 | 246-247 |
| 44 | 5-CH₃-2-thienyl | 3 | HBr 2:1 | 237-238 |
| 45 | 5-CH₃-2-thienyl | - | HBr 2:1 | 210-211 |
| 46 | 5-Cl-2-thienyl | - | HBr 2:1 | 248-250 |
| 47 | 5-Cl-2-thienyl | 3 | HBr 2:1 | 258-259 |
| 48 | 2-Furyl | 3 | HBr 2:1 | 262-264 |
| 49 | 2-Furyl | - | HBr 2:1 | 182 (d) |
| 50 | 5-Indolyl | - | ox. 1:1 | 268-269 |
| 51 | 2-Benzofuryl | 3 | - | 145-146 |
| 52 | 2-Benzofuryl | - | HBr 2:1 | 303-305 |
| 53 | 2-Pyrrolyl | 3 | HBr 2:1 | 265-266 |
| 54 | 2-Pyrrolyl | - | ox. 1:1 | 95-97 |
| 55 | 2-Benzothienyl | 3 | - | 165-166 |
| 56 | 2-Benzothienyl | - | HBr 2:1 | 311-313 |
| 57 | 3-Furyl | 3 | HBr 2:1 | 291-294 |
| 58 | 3-Furyl | - | HBr 2:1 | 313-315 |
| 59 | 4-OH-3-pyridinyl | - | HBr 2:1 | 268-270 |
| 60 | 3,5-(CH₃)₂-1,2-oxazol-4-yl | - | - | 116-117 |
| 61 | 3,5-(CH₃)₂-1,2-oxazol-4-yl | 3 | HBr 2:1 | 250-252 |
| 62 | 2,4-(CH₃O)₂-pyrimidin-5-yl | - | ox. 1:1 | 70-72 |
| 63 | 4-CH₃-2-thienyl | - | HBr 2:1 | 336-338 |
| 64 | 4-CH₃-2-thienyl | 3 | HBr 2:1 | 284-285 |
| 65 | 1-Dibenzofuryl | - | HBr 2:1 | 188-189 |
| 66 | 1-Dibenzofuryl | 3 | HBr 2:1 | 302-304 |
| 67 | 1-Phenoxathiinyl | 3 | HBr 2:1 | 292-293 |
| 68 | 1-Phenoxathiinyl | - | HBr 1:1 | 200-203 |
| 69 | 8-Quinoleinyl | - | HBr 2:1 | 206-208 |
| 70 | 8-Quinoleinyl | 3 | HBr 2:1 | 309-310 |
| 71 | 3-Benzothienyl | 3 | HBr 2:1 | 222-223 |
| 72 | 3-Benzothienyl | - | ox. 1:1 | 80-82 |

Les composés de la présente invention ont été étudiés quant à leur affinité vis-à-vis des récepteurs nicotiniques contenant la sous unité α₄β₂ selon les méthodes décrites par Anderson et Arneric dans *Eur. J. Pharmacol.* (1994), **253**, 261 et par Hall et coll. dans *Brain Res.* (1993), **600**, 127. On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron^{™} dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C. Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]-cytisine à 1 nM dans un volume final de 500 µl de tampon, en présence ou en absence de composé à tester. On arrête la réaction par filtration sur des filtres Whatman GF/B^{™} préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM; la liaison non spécifique représente 75 à 85% de la liaison totale récupérée sur le filtre. Pour chaque concentration de composé étudié on détermine le pourcentage d'inhibition de la liaison spécifique de [³H]-cytisine, puis on calcule la CI₅₀, concentration de composé qui inhibe 50% de la liaison spécifique.
Les CI₅₀ des composés de l'invention les plus affins se situent entre 0,01 et 10 µM.

Les composés de l'invention ont aussi été étudiés quant à leur affinité vis à vis des récepteurs nicotiniques contenant la sous unité α₇, selon les méthodes décrites par Mark et Collins dans *J. Pharmacol. Exp. Ther.* (1982), **22**, 564 et par Marks et coll. dans *Mol. Pharmacol.* (1986), **30**, 427.
On décapite des rats mâles OFA de 150 à 200 g, on prélève rapidement la totalité du cerveau, on l'homogénéise à l'aide d'un broyeur Polytron^{™} dans 15 volumes d'une solution de sucrose à 0,32 M à 4 °C, puis on le centrifuge à 1000 G pendant 10 min. On élimine le culot et on centrifuge le surnageant à 8000 G pendant 20 min. à 4 °C. On récupère le culot et on l'homogénéise à l'aide d'un broyeur Polytron^{™} dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.
Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]α-bungarotoxine à 1 nM dans un volume final de 250 µl de tampon HEPES 20 mM, polyéthylènimine 0,05%. On arrête la réaction par filtration sur des filtres Whatman GF/C^{™} préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale ; la liaison non spécifique représente environ 60 % de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.
Les CI₅₀ des composés de l'invention les plus affins se situent entre 0,005 et 20 µM.

Les résultats qui précèdent montrent que les composés de l'invention sont des ligands pour les récepteurs nicotiniques. Certains sont sélectifs pour les récepteurs contenant les sous-unités α₇ et d'autres sont mixtes pour les récepteurs du type α₄β₂ et α₇.

Les résultats des essais suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MDI).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des pathologies neurodégénératives aiguës telles que les accidents vasculaires cérébraux et les épisodes hypoxiques cérébraux, ainsi que des pathologies neurodégénératives chroniques telles que la maladie d'Alzheimer et la maladie de Parkinson. Ils peuvent être utilisés dans les cas de pathologies psychiatriques : schizophrénie, dépression, anxiété, attaques de panique, comportements compulsifs et obsessionnels.

Ils peuvent prévenir les symptomes dus au sevrage au tabac, à l'alcool, aux différentes substances induisant une dépendance, telles que cocaïne, LSD, cannabis, benzodiazepines.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,01 à 20 mg de principe actif par kg;<de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylène-glycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé, sous forme d'énantiomère pur ou sous forme de mélange d'énantiomères, répondant à la formule générale (I) 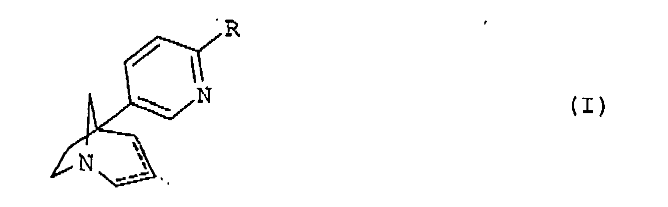 dans laquelle R représente un atome d'halogène ou un groupe (C₃-C₆)cycloalkyle ou un groupe phényle substitué par un ou plusieurs groupes choisis parmi un atome d'halogène, ou un groupe (C₁-C₆) alkyle, (C₁-C₆) alcoxy, nitro, amino, (C₁-C₃)dialkylamino, trifluorométhyle, trifluorométhoxy, cyano, hydroxy, acétyle ou méthylènedioxy, ou un groupe pipéridinyle, ou morpholin-4-yle, ou pyrrolidin-1-yle, ou azétidin-1-yle, ou azépin-1-yle, ou pyridinyle, ou quinoléinyle, ou thiényle, ou pyrazinyle, ou furyle, ou benzofuryle, ou benzothiényle, ou indolyle, ou pyrimidinyle, ou isoxazolyle, ou phénoxazinyle, ou phénoxathiinyle, ou dibenzothiényle, ou dibenzofuryle, ou pyrrolyle ou naphtyle, chacun de ces groupes pouvant éventuellement être substitué par un ou plusieurs groupes choisis parmi les atomes d'halogènes, les groupes (C₁-C₆)alkyle, (C₁-C₆)alcoxy, trifluorométhoxy, trifluorométhyle, nitro, cyano, hydroxy, amino, (C₁-C₃) dialkylamino ou (C₃-C₈) cycloalkylamino, sachant que, parmi les deux liaisons carbone-carbone représentées par 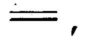 l'une est simple et l'autre peut être simple ou double,
à l'état de base ou de sel d'addition à un acide ; les composés suivants étant exclus :
- 5-(2-bromopyridin-5-yl)-1-azabicyclo[3.2.1]oct-3-ane
- 5-(2-chloropyridin-5-yl)-1-azabicyclo[3.2.1]oct-3-ane
- 5-(2-fluoropyridin-5-yl)-1-azabicyclo[3.2.1] oct-3-ane.

2. Composé selon la revendication 1, **caractérisé en ce que** R représente soit un atome d'halogène ou un groupe phényle substitué par un ou plusieurs groupes choisis parmi les atomes d'halogènes et les groupes (C₁-C₆)alkyle, (C₁-C₆) alcoxy, nitro, amino, trifluorométhyle, cyano, hydroxy, acétyle ou méthylènedioxy, soit un groupe pyridinyle, soit un groupe thiényle, soit un groupe indolyle, soit un groupe pyrimidinyle éventuellement substitué par un ou plusieurs groupes (C₁-C₆)alcoxy.

3. Composé selon la revendication 1, choisi parmi les composes suivants :
- Bromhydrate de 5-[2-(2,4-diméthoxy-5-pyrimidinyl)-pyridin-5-yl]-1-azabicyclo[3.2.1]oct-2-ène (2:1);
- Bromhydrate de 5-[2-(3,4-diméthoxyphényl)pyridiri-5-yl]-1-azabicyclo[3.2.1]oct-3-ène (2:1);
- Bromhydrate de 5-[2-(3,4-diméthoxyphényl)pyridin-5-yl]-1-azabicyclo[3.2.1]octane (2:1);
- Ethanedioate de 5-[2-(2-fluorophényl)pyridin-5-yl]-1-azabicyclo[3.2.1]oct-3-éne (1:1);
- Ethanedioate de 5-[2-(3-méthylphényl)pyridin-5-yl]-1-azabicyclo[3.2.1]oct-3-ène (1:1) ;
- Ethanedioate de 5-[2-(3-fluorophényl)pyridin-5-yl]-1-azabicyclo [3.2.1] oct-3-ène (1:1);
- Ethanedioate de 5-[2-(3-nitrophényl)pyridin-5-yl]-1-azabicyclo[3.2.1]oct-3-ène (1:1);
- Ethanedioate de 5-[2-(3-trifluorométhylphényl)pyridin-5-yl]-1-azabicyclo[3.2.1]oct-3-éne (1:1);
- Ethanedioate de 5-[2-(4-méthylphényl)pyridin-5-yl]-1-azabicyclo [3.2.1] oct-3-ène (1:1);
- Ethanedioate de 5-[2-(3-thiényl)pyridin-5-yl]-1-azabicyclo [3.2.1] oct-3-ène (1:1);
- Ethanedioate de 5-[2-(1,3-benzodioxol-5-yl) pyridin-5-yl]-1-azabicyclo [3.2.1] oct-3-ène (1:1);
- Ethanedioate de 5-[2-(4-chlorophényl) pyridin-5-yl]-1-azabicyelo [3.2.1] oct-3-ène (1:1);
- Ethanedioate de 1-{3-[5-(1-azabicyclo[3.2.1]oct-3-én-5-yl)pyridin-2-yl]phenyl}éthanone (1:1);
- Ethanedioate de 5-[2-(3-pyridinyl)pyridin-5-yl]-1-azabicyclo[3.2.1]oct-3-ène (1:1);
- Ethanedioate de 5-[2-(5-indolyl)pyridin-5-yl]-1-azabicyclo[3.2.1]oct-3-ène (1:1);
- Ethanedioate de 5-[2-(4-méthoxyphényl)pyridin-5-yl]-1-azabicyclo[3.2.1]oct-3-ène (1:1);
- Ethanedioate de 5-[2-(3,5-diméthylphényl)pyridin-5-yl]-1-azabicyclo[3.2.1]oct-3-ène (1:1);
- Ethanedioate de 5-[2-(4-pyridinyl)pyridin-5-yl]-1-azabicyclo[3.2.1]oct-3-ène (1:1);
- Bromhydrate de 5-[2-(4-méthoxyphényl)pyridin-5-yl]-1-azabicyclo[3.2.1]octane (2:1);
- Bromhydrate de 5-[2-(4-méthylphényl)pyridin-5-yl]-1-azabicyclo [3.2.1] octane (2:1);
- Bromhydrate de 5-[2-(3-méthylphényl)pyridin-5-yl]-1-azabicyclo[3.2.1]octane (2:1);
- Bromhydrate de 5-[2-(3,5-diméthylphényl)pyridin-5-yl]-1-azabicyclo[3.2.1]octane (2:1);
- Bromhydrate de 5-[2-(3-fluorophényl)pyridin-5-yll-1-azabicyclo[3.2.1]octane (2:1) ;
- Bromhydrate de 5-[2-(3-thiényl)pyridin-5-yl]-1-azabicyclo [3.2.1] octane (2:1) ;
- Bromhydrate de 5-[2-(1,3-benzodioxol-5-yl)pyridin-5-yl]-1-azabicyclo[3.2.1]octane (2:1) ;
- Bromhydrate de 5-[2-(2-fluorophényl)pyridin-5-yl]-1-azabicyclo[3.2.1]octane (2:1) ;
- Bromhydrate de 5-[2-(3-pyridinyl)pyridin-5-yl]-1-azabicyclo[3.2.1]octane (3:1) ;
- Bromhydrate de 5-[2-(4-pyridinyl)pyridin-5-yl]-1-azabicyclo[3.2.1]octane (2:1) ;
- Bromhydrate de 5-[2-(3-nitrophényl)pyridin-5-yl]-1-azabicyclo[3.2.1]octane (3:1) ;
- Bromhydrate de 5-[2-(3-trifluorophényl)pyridin-5-yl]-1-azabicyclo[3.2.1]octane (2:1) ;
- Bromhydrate de 5-[2-bromopyridin-5-yl]-1-azabicyclo[3.2.1]oct-3-ène (2:1) ;
- Bromhydrate de 5-[2-bromopyridin-5-yl]-1-azabicyclo[3.2.1]oct-2-ène (2:1) ;
- Bromhydrate de 5-[2-(4-pipéridinyl)pyridin-5-yl]-1-azabicyclo[3.2.1]octane (3:1) ;
- Bromhydrate de 5-[2-(3-pipéridinyl)pyridin-5-yl]-l-azabicyclo[3.2.1]octane (3:1) ;
- (+)-5-[2-(4-méthoxyphényl)pyxidin-5-yl]-1-azabicyclo[3.2.1]octane ;
- (-)-5-[2-(4-méthoxyphényl)pyridin-5-yl]-1-azabicyclo[3.2.1]octane ;
- (+)-5-[2-(3-fluorophényl)pyridin-5-yl]-1-azabicyclo[3.2.1]octane ;
- (-)-5-[2-(3-fluorophényl)pyridin-5-yl]-1-azabicyclo[3.2.1]octane ;
- Bromhydrate de 5-[2-(2-thiényl)pyridin-5-yl]-1-azabicyclo[3.2.1]octane (2:1) ;
- Bromhydrate de 5-[2-(2-thiényl)pyridin-5-yl]-1-azabicyclo[3.2.1]oct-3-ène (2:1) ;
- Bromhydrate de 5-[2-(5-méthyl-2-thiényl)pyridin-5-yl]-1-azabicyclo[3.2.1]oct-3-ène (2:1) ;
- Bromhydrate de 5-[2-(5-méthyl-2-thiényl)pyridin-5-yl]-1-azabicyclo [3.2.1] octane (2:1) ;
- Bromhydrate de 5-[2-(5-chloro-2-thiényl) pyridin-5-yl]-1-azabicyclo [3.2.1] octane (2:1) ;
- Bromhydrate de 5-[2-(5-chloro-2-thiényl) pyridin-5-yl]-1-azabicyclo [3.2.1]oct-3-ène (2:,1) ;
- Bromhydrate de 5-[2-(2-furyl) pyridin-5-yl]-1-azabicyclo[3.2.1]oct-3-ène (2:1) ;
- Bromhydrate de 5-[2-(2-furyl) pyridin-5-yl]-1-azabicyclo[3.2.1]octane (2:1) ;
- Ethanedioate de 5-[2-(5-indolyl)pyridin-5-yl]-1-azabicyclo[3.2.1]octane (1:1);
- 5-[2-(2-benzofuryl)pyridin-5-yl]-1-azabicyclo[3.2.1] oct-3-ène ;
- Bromhydrate de 5-[2-(2-benzofuryl)pyridzn-5-yl]-1-azabicyclo[3.2.1]octane (2:1) ;
- Bromhydrate de 5-[2-(2-pyrrolyl)pyridin-5-yl]-1-azabicyclo[3.2.1]oct-3-ène (2:1) ;
- Ethanedioate de 5-[2-(2-pyrrolyl)pyridin-5-yl]-l-azabicyclo[3.2.1]octane (1:1);
- 5-[2-(2-benzothiényl)pyridin-5-yl]-1-azabicyclo[3.2.1]oct-3-ène ;
- Bromhydrate de 5-[2-(2-benzothiènyl)pyridin-5-yl]-1-azabicyclo[3.2.1]octane (2:1) ;
- Bromhydrate de 5-[2-(3-furyl)pyridin-5-yl]-1-azabicyclo[3.2.1]oct-3-ène (2:1) ;
- Bromhydrate de 5-[2-(3-furyl)pyridin-5-yl]-1-azabicyclo[3.2.1]octane (2:1) ;
- Bromhydrate de 5-[2-(4-hydroxy-3-pyridinyl)pyridin-5-yl]-1-azabicyclo[3.2.1]octane (2:1) ;
- 5-[2-(3,5-diméthyl-1,2-oxazol-4-yl)pyridin-5-yl]-1-azabicyclo[3.2.1]octane,
- Bromhydrate de 5-[2-(3,5-diméthyl-1,2-oxazol-4-yl)pyridin-5-yl]-1-azabicyclo[3.2.1]oct-3-ène (2:1);
- Ethanedioate de 5-[2-(2,4-diméthoxypyrimidin-5-yl)pyridin-5-yl]-l-azabicyclo[3.2.1]octane (1:1);
- Bromhydrate de 5-[2-(4-méthyl-2-thiényl)pyridin-5-yl]-1-azabicyclo[3.2.1]octane (2:1)
- Bromhydrate de 5-[2-(4-méthyl-2-thiényl)pyridin-5-yl]-1-azabicyclo [3.2.1]oct--3-ène (2:1) ;
- Bromhydrate de 5-[2-(1-dibenzofuryl)pyridin-5-yl]-1-azabicyclo [3.2.1]octane (2:1) ;
- Bromhydrate de 5-[2-(1-dibenzofuryl)pyridin-5-yl]-1-azabicyclo[3.2.1]oct-3-ène (2:1) ;
- Bromhydrate de 5-[2-(1-phénoxatiinyl)pyridin-5-yl]-1-azabicyclo [3.2.1]oct-3-ène (2:1) ;
- Bromhydrate de 5-[2-(1-phénoxatiinyl)pyridin-5-yl]-1-azabicyclo[3.2.1]octane (1:1) ;
- Bromhydrate de 5-[2-(8-quinoléinyl)pyridin-5-yl]-1-azabicyclo[3.2.1]octane (2:1) ;
- Bromhydrate de 5-[2-(B-quinoléinyl)pyridin-5-y1]-1-azabicyclo [3.2.1] oct-3-éne (2:1) ;
- Bromhydrate de 5-[2-(3-benzothiényl) pyridin-5-y1]-1-azabicyclo[3.2.1]oct-3-ène (2:1) ;
- Ethanedioate de 5-[2-(3-benzothiényl)pyridin-5-yl]-1-azabicyclo[3.2.1]octane (1:1).

4. Composé choisi parmi les composes suivants :
- Bromhydrate de 5-(2-phénylpyridin-5-yl)-1 azabicyclo [3.2.1] oct-3-ène (2:1) ;
- Bromhydrate de 5-(2-phénylpyridin-5-yl)-1-azabicycLo [3.2.1] octane (2:1) ;
- Bromhydrate de 5-(2-phénylpyridin-5-yl)-1 azabicyclo[3.2.1]oct-2-ène (2:1).

5. Médicament **caractérisé en ce qu'**il consiste en un composé selon la revendication 1.

6. Composition pharmaceutique **caractérisée en ce qu'**elle contient une dose efficace d'au moins un composé selon la revendication 1, à l'état de base ou de sel ou de solvat pharmaceutiquement acceptable, et en mélange, le cas échéant, avec des excipients convenables.

## Claims

1. Compound, in the form of pure enantiomer or in the form of mixture of enantiomers, complying with the general formula (I) 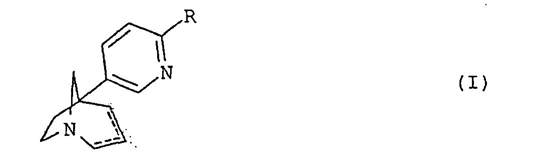 in which R represents a halogen atom or a (C₃-C₆)cycloalkyl group or a phenyl group substituted by one or more groups chosen from a halogen atom, or a (C₁-C₆) alkyl, (C₁-C₆) alkoxy, nitro, amino, (C₁-C₃) - dialkylamino, trifluoromethyl, trifluoromethoxy, cyano, hydroxy, acetyl or methylenedioxy group, or a piperidinyl, or morpholin-4-yl, or pyrrolidin-1-yl, or azetidin-1-yl, or azepin-1-yl, or pyridinyl, or quinolinyl, or thienyl, or pyrazinyl, or furyl, or benzofuryl, or benzothienyl, or indolyl, or pyrimidinyl, or isoxazolyl, or phenoxazinyl, or phenoxathiinyl, or dibenzothienyl, or dibenzofuryl, or pyrrolyl, or naphthyl group, where each of these groups may optionally be substituted by one or more groups chosen from halogen atoms, (C₁-C₆) alkyl, (C₁-C₆) alkoxy, trifluoromethoxy, trifluoromethyl, nitro, cyano, hydroxy, amino, (C₁-C₃)dialkylamino or (C₃-C₈)cycloalkylamino groups, where, of the two carbon-carbon bonds represented by 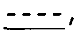 one is single and the other may be single or double, in the form of base or of salt derived from addition to an acid;
with exclusion of the following compounds:
• 5-(2-bromopyridin-5-yl)-1-azabicyclo [3.2.1] oct-3-ane
• 5-(2-chloropyridin-5-yl)-1-azabicyclo[3.2.1]oct-3-ane
• 5-(2-fluoropyridin-5-yl)-1-azabicyclo [3.2.1] oct-3-ane.

2. Compound according to Claim 1, **characterized in that** R represents either a halogen atom or a phenyl group substituted by one or more groups chosen from halogen atoms and (C₁-C₆) alkyl, (C₁-C₆) alkoxy, nitro, amino, trifluoromethyl, cyano, hydroxy, acetyl or methylenedioxy groups, or represents a pyridinyl group, or a thienyl group, or an indolyl group, or a pyrimidinyl group optionally substituted by one or more (C₁-C₆) alkoxy groups.

3. Compound according to Claim 1, chosen from the following compounds:
• hydrobromide of 5-[2-(2,4-dimethoxy-5-pyrimidinyl)-pyridin-5-yl]-1-azabicyclo[3.2.1]oct-2-ene (2:1);
• hydrobromide of 5-[2-(3,4-dimethoxyphenyl)pyridin-5-yl]-1-azabicyclo[3.2.1]oct-3-ene (2:1);
• hydrobromide of 5-[2-(3,4-dimethoxyphenyl)pyridin-5-yl]-1-azabicyclo [3.2.1] octane (2:1) ;
• ethanedioate of 5-[2-(2-fluorophenyl)pyridin-5-yl] - 1-azabicyclo [3.2.1] oct-3-ene (1:1) ;
• ethanedioate of 5- [2- (3-methylphenyl) pyridin-5-yl] - 1-azabicyclo [3.2.1] oct-3-ene (1:1);
• ethanedioate of 5-[2-(3-fluorophenyl) pyridin-5-yl] - 1-azabicyclo[3.2.1]oct-3-ene (1:1) ;
• ethanedioate of 5-[2-(3-nitrophenyl)pyridin-5-yl]-1-azabicyclo [3.2.1] oct-3-ene (1:1) ;
• ethanedioate of 5-[2-(3-trifluoromethylphenyl)pyridin-5-yl]-1-azabicyclo[3.2.1]oct-3-ene (1:1);
• ethanedioate of 5-[2-(4-methylphenyl) pyridin-5-yl]-1-azabicyclo[3.2.1]oct-3-ene (1:1) ;
• ethanedioate of 5-[2-(3-thienyl)pyridin-5-yl]-1-azabicyclo [3.2.1] oct-3-ene (1:1) ;
• ethanedioate of 5-[2-(1,3-benzodioxol-5-yl)pyridin-5-yl]-1-azabicyclo[3.2.1]oct-3-ene (1:1);
• ethanedioate of 5-[2-(4-chlorophenyl)pyridin-5-yl]-1-azabicyclo [3.2.1] oct-3-ene (1:1) ;
• ethanedioate of 1-{3-[5-(1-azabicyclo[3.2.1]oct-3-ene-5-yl)pyridin-2-yl]phenyl}ethanone (1:1);
• ethanedioate of 5-[2-(3-pyridinyl)pyridin-5-yl]-1-azabicyclo[3.2.1]oct-3-ene (1:1) ;
• ethanedioate of 5-[2-(5-indolyl)pyridin-5-yl]-1-azabicyclo[3.2.1]oct-3-ene (1:1) ;
• ethanedioate of 5-[2-(4-methoxyphenyl)pyridin-5-yl]-1-azabicyclo [3.2.1] oct-3-ene (1:1) ;
• ethanedioate of 5-[2-(3,5-dimethylphenyl)pyridin-5-yl]-1-azabicyclo[3.2.1]oct-3-ene (1:1) ;
• ethanedioate of 5-[2-(4-pyridinyl)pyridin-5-yl]-1-azabicyclo[3.2.1]oct-3-ene (1:1) ;
• hydrobromide of 5-[2-(4-methoxyphenyl)pyridin-5-yl]-1-azabicyclo [3.2.1] octane (2:1);
• hydrobromide of 5-[2-(4-methylphenyl) pyridin-5-yl] - 1-azabicyclo [3.2.1] octane (2:1) ;
• hydrobromide of 5-[2-(3-methylphenyl) pyridin-5-yl] - 1-azabicyclo [3.2.1] octane (2:1) ;
• hydrobromide of 5-[2-(3,5-dimethylphenyl)pyridin-5-yl]-1-azabicyclo [3.2.1] octane (2:1);
• hydrobromide of 5- [2-(3-fluorophenyl)pyridin-5-yl] - 1-azabicyclo[3.2.1]octane (2:1) ;
• hydrobromide of 5-[2-(3-thienyl)pyridin-5-yl] -1-azabicyclo [3.2.1] octane (2:1) ;
• hydrobromide of 5-[2-(1,3-benzodioxol-5-yl)pyridin-5-yl] -1-azabicyclo [3.2.1] octane (2:1) ;
• hydrobromide of 5-[2-(2-fluorophenyl)pyridin-5-yl]-1-azabicyclo [3.2.1] octane (2:1) ;
• hydrobromide of 5-[2-(3-pyridinyl)pyridin-5-yl]-1-azabicyclo[3.2.1]octane (3:1) ;
• hydrobromide of 5-[2-(4-pyridinyl)pyridin-5-yl]-1-azabicyclo [3.2.1]octane (2:1) ;
• hydrobromide of 5-[2-(3-nitrophenyl)pyridin-5-yl]-1-azabicyclo[3.2.1]octane (3:1) ;
• hydrobromide of 5-[2-(3-trifluorophenyl)pyridin-5-yl] - 1-azabicyclo [3.2.1] octane (2:1) ;
• hydrobromide of 5-[2-bromopyridin-5-yl]-1-azabicyclo-[3.2.1] oct-3-ene (2:1);
• hydrobromide of 5-[2-bromopyridin-5-yl]-1-azabicyclo-[3.2.1]oct-2-ene (2:1);
• hydrobromide of 5-[2-(4-piperidinyl)pyridin-5-yl]-1-azabicyclo [3.2.1] octane (3:1);
• hydrobromide of 5-[2-(3-piperidinyl)pyridin-5-yl]-1-azabicyclo[3.2.1]octane (3:1) ;
• (+) -5- [2-(4-methoxyphenyl)pyridin-5-yl]-1-azabicyclo-[3.2.1]octane;
• (-)-5-[2-(4-methoxyphenyl)pyridin-5-yl]-1-azabicyclo-[3.2.1]octane;
• (+)-5-[2-(3-fluorophenyl)pyridin-5-yl]-1-azabicyclo-[3.2.1]octane;
• (-)-5-[2-(3-fluorophenyl) pyridin-5-yl]-1-azabicyclo-[3.2.1]octane;
• hydrobromide of 5-[2-(2-thienyl)pyridin-5-yl]-1-azabicyclo [3.2.1] octane (2:1) ;
• hydrobromide of 5-[2-(2-thienyl)pyridin-5-yl]-1-azabicyclo[3.2.1]oct-3-ene (2:1) ;
• hydrobromide of 5-[2-(5-methyl-2-thienyl)pyridin-5-yl]-1-azabicyclo [3.2.1] oct-3-ene (2:1);
• hydrobromide of 5-[2-(5-methyl-2-thienyl)pyridin-5-yl]-1-azabicyclo [3.2.1] octane (2:1);
• hydrobromide of 5-[2-(5-chloro-2-thienyl)pyridin-5-yl]-1-azabicyclo [3.2.1] octane (2:1);
• hydrobromide of 5-[2-(5-chloro-2-thienyl)pyridin-5-yl]-1-azabicyclo [3.2.1] oct-3-ene (2:1);
• hydrobromide of 5-[2-(2-furyl)pyridin-5-yl]-1-azabicyclo [3.2.1]oct-3-ene (2:1);
• hydrobromide of 5-[2-(2-furyl)pyridin-5-yl]-1-azabicyclo [3.2.1]octane (2:1) ;
• ethanedioate of 5-[2-(5-indolyl)pyridin-5-yl]-1-azabicyclo [3.2.1]octane (1:1);
• 5-[2-(2-benzofuryl)pyridin-5-yl]-1-azabicyclo[3.2.1]-oct-3-ene;
• hydrobromide of 5-[2-(2-benzofuryl)pyridin-5-yl]- 1-azabicyclo[3.2.1]octane (2:1) ;
• hydrobromide of 5-[2-(2-pyrrolyl)pyridin-5-yl]-1-azabicyclo [3.2.1]oct-3-ene (2:1);
• ethanedioate of 5-[2-(2-pyrrolyl)pyridin-5-yl]-1-azabicyclo [3.2.1]octane (1:1);
• 5-[2-(2-benzothienyl)pyridin-5-yl]-1-azabicyclo-[3.2.1]oct-3-ene;
• hydrobromide of 5-[2-(2-benzothienyl)pyridin-5-yl]-1-azabicyclo[3.2.1]octane (2:1);
• hydrobromide of 5-[2-(3-furyl)pyridin-5-yl]-1-azabicyclo[3.2.1]oct-3-ene (2:1) ;
• hydrobromide of 5-[2-(3-furyl)pyridin-5-yl]-1-azabicyclo [3.2.1]octane (2:1) ;
• hydrobromide of 5-[2-(4-hydroxy-3-pyridinyl)pyridin-5-yl]-1-azabicyclo [3.2.1]octane (2:1) ;
• 5-[2-(3,5-dimethyl-1,2-oxazol-4-yl)pyridin-5-yl]-1-azabicyclo [3.2.1]octane;
• hydrobromide of 5-[2-(3,5-dimethyl-1,2-oxazol-4-yl)-pyridin-5-yl]-1-azabicyclo [3.2.1] oct-3-ene (2:1);
• ethanedioate of 5-[2-(2,4-dimethoxypyrimidin-5-yl)-pyridin-5-yl]-1-azabicyclo[3.2.1]octane (1:1);
• hydrobromide of 5-[2-(4-methyl-2-thienyl)pyridin-5-yl]-1-azabicyclo [3.2.1]octane (2:1) ;
• hydrobromide of 5-[2-(4-methyl-2-thienyl)pyridin-5-yl] -1-azabicyclo [3.2.1] oct-3-ene (2:1) ;
• hydrobromide of 5-[2-(1-dibenzofuryl)pyridin-5-yl]-1-azabicyclo [3.2.1]octane (2:1) ;
• hydrobromide of 5-[2-(1-dibenzofuryl)pyridin-5-yl]-1-azabicyclo[3.2.1]oct-3-ene (2:1) ;
• hydrobromide of 5-[2-(1-phenoxathiinyl)pyridin-5-yl]-1-azabicyclo[3.2.1]oct-3-ene (2:1);
• hydrobromide of 5-[2-(1-phenoxathiinyl)pyridin-5-yl]-1-azabicyclo [3.2.1]octane (1:1) ;
• hydrobromide of 5-[2-(8-quinolinyl)pyridin-5-yl]-1-azabicyclo[3.2.1]octane (2:1) ;
• hydrobromide of 5-[2-(8-quinolinyl)pyridin-5-yl]-1-azabicyclo [3.2.1]oct-3-ene (2:1) ;
• hydrobromide of 5-[2-(3-benzothienyl)pyridin-5-yl]-1-azabicyclo [3.2.1]oct-3-ene (2:1);
• ethanedioate of 5-[2-(3-benzothienyl)pyridin-5-yl]-1-azabicyclo [3.2.1]octane (1:1).

4. Compound chosen from the following compounds:
• hydrobromide of 5-(2-phenylpyridin-5-yl)-1-azabicyclo-[3.2.1]oct-3-ene (2:1);
• hydrobromide of 5-(2-phenylpyridin-5-yl)-1-azabicyclo-[3.2.1]octane (2:1);
• hydrobromide of 5-(2-phenylpyridin-5-yl)-1-azabicyclo-[3.2.1] oct-2-ene (2:1).

5. Medicament **characterized in that** it consists of a compound according to Claim 1.

6. Pharmaceutical composition **characterized in that** it comprises an effective dose of at least one compound according to Claim 1, in the form of base or of pharmaceutically acceptable salt or solvate, and as a mixture, where appropriate, with suitable excipients.

## Patentansprüche

1. In Form eines reinen Enantiomers oder eines Enantiomerengemischs vorliegende Verbindung der allgemeinen Formel (I) 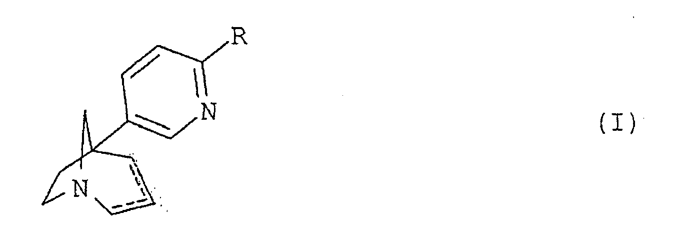 worin R für ein Halogenatom, eine (C₃-C₆)-Cycloalkylgruppe, eine durch eine oder mehrere unter einem Halogenatom oder einer (C₁-C₆)-Alkyl-, (C₁-C₆) -Alkoxy-, Nitro-, Amino-, (C₁-C₃) - Dialkylamino-, Trifluormethyl-, Trifluormethoxy-, Cyano-, Hydroxy-, Acetyl- oder Methylendioxygruppe ausgewählte Gruppen substituierte Phenylgruppe oder eine Piperidyl-, Morpholin-4-yl-, Pyrrolidin-1-yl-, Azetidin-1-yl-, Azepin-1-yl, Pyridinyl-, Chinoleinyl-, Thienyl-, Pyrazinyl-, Furyl-, Benzofuryl-, Benzothienyl-, Indolyl-, Pyrimidinyl-, Isoxazolyl-, Phenoxazinyl-, Phenoxathiinyl-, Dibenzothienyl-, Dibenzofuryl-, Pyrollyl- oder Naphthylgruppe, wobei jede dieser Gruppen gegebenenfalls durch eine oder mehrere unter Halogenatomen oder (C₁-C₆) -Alkyl-, (C₁-C₆)-Alkoxy- , Trifluormethoxy-, Trifluormethyl-, Nitro-, Cyano-, Hydroxy-, Amino-, (C₁-C₃) -Dialkylamino- oder (C₃-C₈)-Cycloalkylamino ausgewählte Gruppen substituiert sein kann, steht,
mit der Maßgabe, daß von den beiden durch 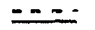 dargestellten Kohlenstoff-Kohlenstoff-Bindungen eine einfach ist und die andere einfach oder doppelt sein kann,
in Basen- oder Säureadditionssalzform;
unter Ausschluß der folgenden Verbindungen:
- 5-(2-Brompyridin-5-yl)-1-azabicyclo[3.2.1]oct-3-an
- 5-(2-Chlorpyridin-5-yl)-1-azabicyclo[3.2.1]oct-3-an
- 5-(2-Fluorpyridin-5-yl)-1-azabicyclo[3.2.1]oct-3-an.

2. Verbindung nach Anspruch 1, **dadurch gekennzeichnet, daß** R für ein Halogenatom, eine durch eine oder mehrere unter Halogenatomen und (C₁-C₆) - Alkyl-, (C₁-C₆) -Alkoxy-, Nitro-, Amino-, Trifluormethyl-, Cyano-, Hydroxy-, Acetyl- oder Methylendioxygruppen ausgewählte Gruppen substituierte Phenylgruppe, eine Pyridinylgruppe, eine Thienylgruppe, eine Indolylgruppe oder eine gegebenenfalls durch eine oder mehrere (C₁-C₆)-Alkoxygruppen substituierte Pyrimidinylgruppe steht.

3. Verbindung nach Anspruch 1, ausgewählt unter den folgenden Verbindungen:
- 5-[2-(2,4-Dimethoxy-5-pyrimidinyl)pyridin-5-yl]-1-azabicyclo[3.2.1]oct-2-en-hydrobromid (1:2);
- 5-[2-(3,4-Dimethoxyphenyl)pyridin-5-yl]-1-azabicyclo[3.2.1]oct-3-en-hydrobromid (1:2);
- 5-[2-(3,4-Dimethoxyphenyl)pyridin-5-yl]-1-azabicyclo[3.2.1]octan-hydrobromid (1:2);
- 5-[2-(2-Fluorphenyl)pyridin-5-yl]-1-azabicyclo-[3.2.1]oct-3-en-Ethandisäuresalz (1:1);
- 5-[2-(3-Methylphenyl)pyridin-5-yl]-1-azabicyclo-[3.2.1]oct-3-en-Ethandisäuresalz (1:1);
- 5-[2-(3-Fluorphenyl)pyridin-5-yl]-1-azabicyclo-[3.2.1]oct-3-en-Ethandisäuresalz (1:1);
- 5-[2-.(3-Nitrophenyl)pyridin-5-yl]-1-azabicyclo-[3.2.1]oct-3-en-Ethandisäuresalz (1:1);
- 5-[2-(3-Trifluormethylphenyl)pyridin-5-yl]-1-azabicyclo[3.2.1]oct-3-en-Ethandisäuresalz (1:1);
- 5-[2-(4-Methylphenyl)pyridin-5-yl]-1-azabicyclo-[3.2.1]oct-3-en-Ethandisäuresalz (1:1);
- 5-[2-(3-Thienyl)pyridin-5-yl]-1-azabicyclo-[3.2.1]oct-3-en-Ethandisäuresalz (1:1);
- 5-[2-(1,3-Benzodioxol-5-yl)pyridin-5-yl]-1-azabicyclo[3.2.1]oct-3-en-Ethandisäuresalz (1:1);
- 5-[2-(4-Chlorphenyl)pyridin-5-yl]-1-azabicyclo-[3.2.1]oct-3-en-Ethandisäuresalz (1:1);
- 1-{3-[5-(1-Azabicyclo[3.2.1]oct-3-en-5-yl)-pyridin-2-yl]phenyl}ethanon-Ethandisäuresalz (1:1);
- 5-[2-(3-Pyridinyl)pyridin-5-yl]-1-azabicyclo-[3.2.1]oct-3-en-Ethandisäuresalz (1:1);
- 5-[2-(5-Indolyl)pyridin-5-yl]-1-azabicyclo-[3.2.1]oct-3-en-Ethandisäuresalz (1:1);
- 5- [2- (4-Methoxyphenyl)pyridin-5-yl] -1-azabicyclo[3.2.1]oct-3-en-Ethandisäuresalz (1:1);
- 5-[2-(3,5-Dimethylphenyl)pyridin-5-yl]-1-azabicyclo[3.2.1]oct-3-en-Ethandisäuresalz (1:1);
- 5-[2-(4-Pyridinyl)pyridin-5-yl]-1-azabicyclo-[3.2.1]oct-3-en-Ethandisäuresalz (1:1);
- 5-[2-(4-Methoxyphenyl) pyridin-5-yl]-1-azabicyclo[3.2.1]octan-hydrobromid (1:2);
- 5-[2-(4-Methylphenyl)pyridin-5-yl]-1-azabicyclo-[3.2.1]octan-hydrobromid (1:2);
- 5-[2-(3-Methylphenyl)pyridin-5-yl]-1-azabicyclo-[3.2.1]octan-hydrobromid (1:2);
- 5-[2-(3,5-Dimethylphenyl)pyridin-5-yl]-1-azabicyclo [3.2.1] octan-hydrobromid (1:2);
- 5-[2-(3-Fluorphenyl)pyridin-5-yl]-1-azabicyclo-[3.2.1]octan-hydrobromid (1:2);
- 5-[2-(3-Thienyl)pyridin-5-yl]-1-azabicyclo-[3.2.1]octan-hydrobromid (1:2);
- 5-[2-(1,3-Benzodioxol-5-yl)pyridin-5-yl]-1-azabicyclo[3.2.1]octan-hydrobromid (1:2);
- 5-[2-(2-Fluorphenyl)pyridin-5-yl]-1-azabicyclo-[3.2.1] octan-hydrobromid (1:2);
- 5-[2-(3-Pyridinyl)pyridin-5-yl]-1-azabicyclo-[3.2.1] octan-hydrobromid (1:3);
- 5-[2-(4-Pyridinyl)pyridin-5-yl]-1-azabicyclo-[3.2.1] octan-hydrobromid (1:2);
- 5-[2-(3-Nitrophenyl) pyridin-5-yl]-1-azabicyclo-[3.2.1] octan-hydrobromid (1:3);
- 5- [2- (3-Trifluorphenyl) pyridin-5-yl] -1-azabicyclo[3.2.1]octan-hydrobromid (1:2);
- 5-[2-Brompyridin-5-yl]-1-azabicyclo [3.2.1] oct-3-en-hydrobromid (1:2);
- 5-[2-Brompyridin-5-yl]-1-azabicyclo [3.2.1] oct-2-en-hydrobromid (1:2);
- 5-[2-(4-Piperidinyl)pyridin-5-yl]-1-azabicyclo-[3.2.1] octan-hydrobromid (1:3);
- 5-[2-(3-Piperidinyl)pyridin-5-yl]-1-azabicyclo-[3.2.1]octan-hydrobromid (1:3);
- (+)-5-[2-(4-Methoxyphenyl)pyridin-5-yl]-1-azabicyclo[3.2.1]octan ;
- (-) -5- [2-(4-Methoxyphenyl) pyridin-5-yl] -1-azabicyclo [3.2.1] octan;
- (+)-5-[2-(3-Fluorphenyl)pyridin-5-yl]-1-azabicyclo [3.2.1] octan;
- (-)-5-[2-(3-Fluorphenyl)pyridin-5-yl]-1-azabicyclo[3.2.1]octan ;
- 5-[2-(2-Thienyl)pyridin-5-yl]-1-azabicyclo-[3.2.1]octan-hydrobromid (1:2);
- 5-[2-(2-Thienyl)pyridin-5-yl]-1-azabicyclo-[3.2.1]oct-3-en-hydrobromid (1:2);
- 5-[2-(5-Methyl-2-thienyl)pyridin-5-yl]-1-azabicyclo[3.2.1]oct-3-en-hydrobromid (1:2);
- 5-[2-(5-Methyl-2-thienyl)pyridin-5-yl]-1-azabicyclo[3.2.1]octan-hydrobromid (1:2);
- 5-[2-(5-Chlor-2-thienyl)pyridin-5-yl]-1-azabicyclo[3.2.1]octan-hydrobromid (1:2);
- 5-[2-(5-Chlor-2-thienyl)pyridin-5-yl]-1-azabicyclo[3.2.1]oct-3-en-hydrobromid (1:2);
- 5-[2-(2-Furyl)pyridin-5-yl]-1-azabicyclo[3.2.1]-oct-3-en-hydrobromid (1:2);
- 5-[2-(2-Furyl)pyridin-5-yl]-1-azabicyclo[3.2.1]-octan-hydrobromid (1:2);
- 5-[2-(5-Indolyl)pyridin-5-yl]-1-azabicyclo-[3.2.1]octan-Ethandisäuresalz (1:1);
- 5-[2-(2-Benzofuryl)pyridin-5-yl]-1-azabicyclo-[3.2.1] oct-3-en;
- 5-[2-(2-Benzofuryl)pyridin-5-yl]-1-azabicyclo-[3.2.1]octan-hydrobromid (1:2);
- 5-[2-(2-Pyrrolyl)pyridin-5-yl]-1-azabicyclo-[3.2.1]oct-3-en-hydrobromid (1:2);
- 5-[2-(2-Pyrrolyl)pyridin-5-yl]-1-azabicyclo-[3.2.1]octan-Ethandisäuresalt (1:1);
- 5-[2-(2-Benzothienyl)pyridin-5-yl]-1-azabicyclo-[3.2.1] oct-3-en;
- 5-[2-(2-Benzothienyl)pyridin-5-yl]-1-azabicyclo-[3.2.1]octan-hydrobromid (1:2);
- 5-[2-(3-Furyl)pyridin-5-yl]-l-azabicyclo[3.2.1]-oct-3-en-hydrobromid (1:2);
- 5- [2- (3-Furyl) pyridin-5-yl] -1-azabicyclo [3.2.1]- octan-hydrobromid (1:2);
- 5-[2-(4-Hydroxy-3-pyridinyl)pyridin-5-yl]-1-azabicyclo[3.2.1]octan-hydrobromid (1:2);
- 5-[2-(3,5-Dimethyl-1,2-oxazol-4-yl)pyridin-5-yl] -1-azabicyclo [3.2.1] octan;
- 5-[2-(3,5-Dimethyl-1,2-oxazol-4-yl)pyridin-5-yl]-1-azabicyclo[3.2.1]oct-3-en-hydrobromid (1:2);
- 5-[2-(2,4-Dimethoxypyrimidin-5-yl)pyridin-5-yl]-1-azabicyclo [3.2.1] octan-Ethandisäuresalz (1:1);
- 5-[2-(4-Methyl-2-thienyl)pyridin-5-yl]-1-azabicyclo[3.2.1]octan-hydrobromid (1:2);
- 5-[2-(4-Methyl-2-thienyl)pyridin-5-yl]-1-azabicyclo [3.2.1] oct-3-en-hydrobromid (1:2);
- 5-[2-(1-Dibenzofuryl)pyridin-5-yl]-1-azabicyclo-[3.2.1] octan-hydrobromid (1:2);
- 5-[2-(1-Dibenzofuryl)pyridin-5-yl]-1-azabicyclo-[3.2.1]oct-3-en-hydrobromid (1:2);
- 5-[2-(1-Phenoxathiinyl)pyridin-5-yl]-1-azabicyclo[3.2.1]oct-3-en-hydrobromid (1:2);
- 5-[2-(1-Phenoxathiinyl)pyridin-5-yl]-1-azabicyclo[3.2.1]octan-hydrobromid (1:1);
- 5-[2-(8-Chinoleinyl)pyridin-5-yl]-1-azabicyclo-[3.2.1]octan-hydrobromid (1:2);
- 5-[2-(8-Chinoleinyl)pyridin-5-yl]-1-azabicyclo-[3.2.1]oct-3-en-hydrobromid (1:2);
- 5-[2-(3-Benzothienyl)pyridin-5-yl]-1-azabicyclo-[3.2.1] oct-3-en-hydrobromid (1:2);
- 5-[2-(3-Benzothienyl)pyridin-5-yl]-1-azabicyclo-[3.2.1] octan-Ethandisäuresalz (1:1).

4. Verbindung, ausgewählt unter den folgenden Verbindungen:
- 5- [2-Phenylpyridin-5-yl] -1-azabicyclo [3.2.1] oct-3-en-hydrobromid (1:2);
- 5- [2-Phenylpyridin-5-yl] -1-azabicyclo [3.2.1] - octan-hydrobromid (1:2);
- 5- [2-Phenylpyridin-5-yl] -1-azabicyclo [3.2.1] oct-2-en-hydrobromid (1:2);

5. Medikament, **dadurch gekennzeichnet, daß** es aus einer Verbindung gemäß Anspruch 1 besteht.

6. Pharmazeutische Zusammensetzung, **dadurch gekennzeichnet, daß** sie eine wirksame Dosis mindestens einer Verbindung gemäß Anspruch 1 in Basen- oder Salzform oder pharmazeutisch unbedenklicher Solvatform, gegebenenfalls in Abmischung mit geeigneten Hilfsstoffen, enthält.
